# EUROPÄISCHE PATENTSCHRIFT

(11) **EP 4 472 819 B1**
(45) Veröffentlichungstag und Bekanntmachung des Hinweises auf die Patenterteilung: **25.11.2026**
(21) Anmeldenummer: 23701479.0
(22) Anmeldetag: 22.01.2023
(51) Int. Cl.: B29B 7/58, B29B 7/74, B29B 9/12, B29B 9/16, B29C 48/00, B29C 48/04, B29C 48/05, B29C 48/345, B29C 48/76, B29B 7/42, B29B 7/48, B29B 9/06, B29B 13/00, B29B 17/02

(54) **VORRICHTUNG UND VERFAHREN ZUR WIEDERAUFBEREITUNG VON ZU FLAKES ZERKLEINERTEN UND GEWASCHENEN POST-CONSUMER KUNSTSTOFFABFÄLLEN**
DEVICE AND METHOD FOR RECYCLING POST-CONSUMER PLASTIC WASTE CRUSHED AND WASHED TO FORM FLAKES
DISPOSITIF ET PROCÉDÉ DE RETRAITEMENT DES DÉCHETS EN MATIÈRE PLASTIQUE POST-CONSOMMATION BROYÉS EN FLOCONS ET LAVÉS

(30) Priorität: 02.02.2022 EP 22154679
(43) Veröffentlichungstag der Anmeldung: 11.12.2024
(73) Patentinhaber: Starlinger & Co Gesellschaft m.b.H., 1060 Wien (AT)
(72) Erfinder: GUGGENBERGER, Jacqueline, 2331 Vösendorf (AT)
(74) Vertreter: Schwarz & Partner Patentanwälte GmbH
(86) Internationale Anmeldenummer: PCT/EP2023/051457
(87) Internationale Veröffentlichungsnummer: WO 2023/148025

(56) Entgegenhaltungen:
- EP-A1- 2 995 436
- EP-B1- 2 507 022
- DD-A1- 231 029
- US-A1- 2009 218 052
- US-B2- 9 550 132
- WIEDMANN W [DE] ET AL: "WIRTSCHAFTLICHER EINSATZ VON ZWEIWELLENEXTRUDERN BEIM RECYCLING", KUNSTSTOFFE, CARL HANSER VERLAG, MÜNCHEN [DE], vol. 82, no. 10, 1 October 1992 (1992-10-01), pages 934 - 938, XP000310147, ISSN: 0023-5563
- N.N.: "Conceptual Ozone Laundry Application Diagram", 7 June 2019 (2019-06-07), XP055642963, Retrieved from the Internet <URL:http://www.absoluteozone.com/assets/laundry.pdf> [retrieved on 20191115]

## Beschreibung

Die Erfindung betrifft eine Vorrichtung und ein Verfahren zur Wiederaufbereitung von Flakes aus zerkleinerten und gewaschenen Post-Consumer Kunststoffabfällen.

Die Wiederverwendung von Kunststoffmaterialien spielt eine entscheidende Rolle im Sinne einer nachhaltigen Kreislaufwirtschaft. Um dies zu ermöglichen, ist es nötig, insbesondere Post-Consumer Kunststoffabfälle von geruchsintensiven und infektiösen Kontaminationen zu befreien. Bei der Verwendung von Recyclingkunststoff als Lebensmittelverpackung werden besonders hohe Reinheitsanforderungen an den wiederaufbereiteten Kunststoff gestellt. Außerdem muss sichergestellt werden, dass Geschmack, Geruch und Konsistenz der Lebensmittel durch deren aus Recyclingware gewonnener Verpackung nicht beeinträchtigt werden.

Üblicherweise erfolgt eine Aufbereitung von Post-Consumer Kunststoffabfällen, indem diese in einem ersten Schritt zu Flakes/Häckselgut zerkleinert werden und anschließend einem Waschprozess zugeführt werden. Vorzugsweise wird der Waschprozess in einer Heißwäsche unter Zugabe von Natronlauge oder anderen Waschadditiven realisiert, um die Oberfläche zu reinigen.

Ist für die weitere Verwendung eine bestimmte Farbreinheit gefordert, so empfiehlt sich eine optische Vorsortierung der Post-Consumer Kunststoffabfälle vor ihrer Zerkleinerung, gegebenenfalls auch in Kombination mit einer Sortierung nach dem Waschprozess. Die so hergestellten Flakes können im Anschluss daran zu Regranulaten extrudiert werden. Diese Regranulate werden anschließend mittels Heißluft für kürzere Zeit (Stunden bis Tage) oder mit Umgebungsluft für längere Zeit (Tage bis Wochen) belüftet, um den Geruch des Regranulates zu reduzieren.

Um einen entscheidenden Beitrag zu einer positiven Ökobilanz leisten zu können, muss die Aufbereitung von Kunststoffabfällen und deren Aufwertung zu neuwertigen Produkten möglichst Energie sparend, Ressourcen und Umwelt schonend sowie kostengünstig im Vergleich zur Herstellung von Neuware erfolgen. Mit herkömmlichen Entgasungsanlagen ist die Dekontamination von Post-Consumer Abfällen besonders energie- und zeitintensiv, da die verunreinigten Pellets mehrere Stunden einem Heißluftstrom ausgesetzt werden oder unter Vakuum verweilen. Aus der Schrift EP 2507022 B1 sind ein Verfahren und eine Anlage zur Dekontamination von Kunststoffabfällen bekannt, bei denen Flakes zu Pellets extrudiert werden und die solcherart gewonnenen Pellets einer Geruchsreduzierung unterzogen werden, indem Heißluft durch eine Geruchsentfernungseinheit geblasen wird, in der die Pellets verweilen.

Aufgabe der vorliegenden Erfindung ist es, einen effizienten und gleichzeitig umweltschonenden Prozess zur Aufbereitung von Post-Consumer Abfällen zu bieten, welcher geruchsneutrales Regranulat liefern kann. Im Idealfall kann mit der vorliegenden Erfindung die Reinigungseffizienz in der Aufbereitung von Post-Consumer Kunststoffen so weit verbessert werden, dass die Aufbereitung lebensmitteltaugliches Regranulat liefert.

Aus dem Dokument CN 108641398 ist es bekannt, Kunststoffflaschen in einer speziellen Waschlösung zu waschen und in einem Mixer bis 180-220 °C mit Ozon zu versetzen. Anschließend kann der Kunststoff granuliert werden.

Das Dokument CA 601047 beschreibt die Behandlung von PET-Stapelfasern und PET-Film mit Ozon bei Raumtemperatur, um eine Bedruckung zu ermöglichen. Es wurde festgestellt, dass das PET-Material dabei biegsam und robust bleibt, wobei ein Bleicheffekt beobachtet wurde. Die Ozonbehandlung wurde auch mit einem Wasserdampf/Ozon-Gemisch durchgeführt. In Versuchen wurde festgestellt, dass eine Behandlung des PET-Materials bei 180-230 °C keine negativen Auswirkungen auf das Material hatte. Bei noch höheren Temperaturen zersetzt sich aber das Ozon zu Sauerstoff.

Im Dokument EP 0602505 wird ein Ozon-bildender UV-Licht-Strahler zur Behandlung von Flüssigkeiten zur Schadstoffreduktion beschrieben.

Das Dokument EP 3705252 A1 beschreibt ein Verfahren zur Herstellung eines Plastikmaterials aus Kunststoffabfällen. Dieses Verfahren umfasst das Auswählen von Industrie- oder Post-Consumer Kunststoffabfällen, das Vermahlen der Abfälle, die Behandlung der Abfälle zur Reduzierung der von den Abfällen erzeugten Gerüche, die Behandlung der Abfälle zur Reduzierung der mikrobiellen Belastung, das Tempern der Abfälle in Wasser, die Regranulierung der Abfälle und die Entfeuchtung des Granulats. Zur Geruchsreduzierung und zur Reduzierung der mikrobiellen Belastung kann eine nicht näher erläuterte Ozonisierung angewandt werden.

Nachteilig an diesem bekannten Verfahren ist, dass die Behandlung zur Reduzierung der Gerüche und mikrobiellen Belastung nur an den gemahlenen Abfällen durchgeführt wird. Es ist aber bekannt, dass insbesondere apolare Verbindungen in den Kunststoff migrieren, siehe z.B. Resources, Conservation & Recycling 161 (2020) "Development and application of an analytical method to quantify odour removal in plastic waste recycling processes". Beim Regranulieren sowie bei der Weiterverarbeitung von Regranulat können Verunreinigungen freigesetzt werden, die ihrerseits eine Geruchsbelastung darstellen und daher die Verwendung des Regranulats für höherwertige Anwendungen, insbesondere für die Herstellung von Verpackungen für Lebensmittel, verhindern.

Das Dokument EP 2995436 A1 offenbart ein Verfahren und eine Vorrichtung zur Wiederaufbereitung von Flakes aus zerkleinerten und gewaschenen Post-Consumer Kunststoffabfällen. Die Vorrichtung umfasst eine Vorbehandlungseinheit, einen stromabwärts von der Vorbehandlungseinheit angeordneten Aufschmelzextruder, einen stromabwärts vom Aufschmelzextruder angeordneten Entgasungsextruder und eine stromabwärts vom Entgasungsextruder angeordnete Granuliervorrichtung zur Granulierung der Kunststoffschmelze. Die Vorrichtung weist keine Geruchsentfernungseinheit auf, um das Granulat einer Geruchsentfernung zu unterziehen.

Das Dokument US 2009/218052 A1 offenbart ein Trocknungssystem zur Verwendung in einem Polyesterpolymerherstellverfahren. Das Trocknungssystem kann in einem Nicht-Festphasen-Polymerisationsverfahren verwendet werden, um die Polymerpartikel vor ihrer Kristallisation zu trocknen.

Das Dokument US 9550132 B2 beschreibt ein Verfahren zur Behandlung von Polyolefingranulat in einem Behandlungsbehälter, das die folgenden Schritte umfasst: Bereitstellen eines Granulatbetts in Flüssigkeit in dem Behandlungsbehälter; Abziehen eines ersten Flüssigkeitsstroms aus dem Behandlungsbehälter, wobei der erste Flüssigkeitsstrom kohlenwasserstoffhaltige Verbindungen enthält; Einleiten eines ersten Dampfstroms in den Behandlungsbehälter; Abziehen eines zweiten Dampfstroms aus dem Behandlungsbehälter, wobei der zweite Dampfstrom flüchtige Kohlenwasserstoffverbindungen enthält; Rückgewinnung des Granulats aus dem Behandlungsgefäß, wobei der erste Dampfstrom eine Temperatur von Tb bis Tb+10°C hat, wobei Tb der Siedepunkt der Flüssigkeit bei dem angewandten Druck ist.

Es besteht daher nach wie vor ein Bedarf an einem effizienten und gleichzeitig umweltschonenden Prozess zur Aufbereitung von Post-Consumer Abfällen, welcher geruchsneutrales Regranulat liefern kann, sowie an Vorrichtungen zur Durchführung dieses Prozesses.

Die vorliegende Erfindung löst die gestellte Aufgabe durch Bereitstellen einer Vorrichtung zur Wiederaufbereitung von Flakes aus zerkleinerten und gewaschenen Post-Consumer Kunststoffabfällen mit den Merkmalen des Anspruchs 1 und durch Bereitstellen eines Verfahrens zur Wiederaufbereitung von Flakes aus zerkleinerten und gewaschenen Post-Consumer Kunststoffabfällen mit den Merkmalen des Anspruchs 7. Ausführungsformen der Erfindung sind in den Unteransprüchen, der Beschreibung und den Zeichnungen definiert.

Die erfindungsgemäße Vorrichtung zur Wiederaufbereitung von Flakes aus zerkleinerten und gewaschenen Post-Consumer Kunststoffabfällen umfasst:
eine Vorbehandlungseinheit zur Trocknung und Homogenisierung der Flakes aus zerkleinerten und gewaschenen Post-Consumer Kunststoffabfällen,
einen stromabwärts von der Vorbehandlungseinheit angeordneten Aufschmelzextruder zum Aufschmelzen der in der Vorbehandlungseinheit getrockneten und homogenisierten Kunststoffabfälle,
einen stromabwärts vom Aufschmelzextruder angeordneten Entgasungsextruder mit einem Anschluss an eine Vakuumquelle zum Entgasen der Kunststoffschmelze,
eine stromabwärts vom Entgasungsextruder angeordnete Granuliervorrichtung zur Granulierung der Kunststoffschmelze,
eine stromabwärts von der Granuliervorrichtung angeordnete Geruchsentfernungseinheit, um das Granulat einer Geruchsentfernung zu unterziehen,
wobei die Geruchsentfernungseinheit einen Prozessgaszulauf und einen Gasablauf zum Austragen eines Abgasstroms aufweist, wobei der Prozessgaszulauf der Geruchsentfernungseinheit mit einer Ozonquelle oder einer Ozonerzeugungsvorrichtung verbunden ist, wodurch der Geruchsentfernungseinheit mit Ozon angereichertes Prozessgas zuführbar ist.

Somit kann einerseits eine Ozonquelle (z.B. in Form einer Gasflasche, gefüllt mit einem mit Ozon angereicherten Gas, z.B. mit Ozon angereicherter Druckluft) an die Geruchsentfernungseinheit angeschlossen werden. Der Begriff "Ozonquelle" bedeutet nicht, dass diese Quelle reines Ozon bereitstellt. Andererseits kann eine Ozonerzeugungsvorrichtung an die Geruchsentfernungseinheit angeschlossen sein, wobei die Ozonerzeugungsvorrichtung Umgebungsluft als Prozessgas ansaugt oder dem Prozessgaszulauf entnimmt und mit Ozon anreichert, indem sie den in der Luft enthaltenen Sauerstoff unter elektrischer Spannung oder UV-Strahlung zu Ozon reagiert.

Das erfindungsgemäße Verfahren zur Wiederaufbereitung von Flakes aus zerkleinerten und gewaschenen Post-Consumer Kunststoffabfällen umfasst:
das Trocknen und Homogenisieren der Flakes aus zerkleinerten und gewaschenen Post-Consumer Kunststoffabfällen in einem Gasstrom, insbesondere einem Heißgasstrom,
das Aufschmelzen der getrockneten und homogenisierten Kunststoffabfälle zu einer Kunststoffschmelze unter Verwendung eines Aufschmelzextruders,
optional das Ausfiltern von Fremdkörpern aus der Kunststoffschmelze,
das Entgasen der Kunststoffschmelze unter Verwendung eines Entgasungsextruders,
das Granulieren der Kunststoffschmelze,
optional das Trocknen des Granulats,
das Entfernen von Gerüchen aus dem Granulat durch einen Prozessgasstrom, und
das Anreichern des zum Entfernen von Gerüchen aus dem Granulat verwendeten Prozessgasstroms mit Ozon oder das Bereitstellen des Prozessgasstroms als ein mit Ozon angereichertes Gas.

Die vorliegende Erfindung ermöglicht es, Geruchsstoffe zielgerichtet aus Post-Consumer Abfällen zu entfernen. Das Ausmaß an verbleibenden Restgerüchen des erfindungsgemäß erzeugten und behandelten Granulats kann auf verschiedene Arten bestimmt werden. Man kann beispielsweise verschiedene Proben des Granulats in verdünnter oder unverdünnter Luft durch ein Gremium von Testpersonen bewerten lassen, die für diese Aufgabe geschult sind. Alternativ dazu können materialspezifische Indikatoren festgelegt und über die verbleibende Restmenge nach dem erfolgten Geruchsentfernungsprozess bewertet werden. Darüber hinaus lässt sich bei einer gaschromatografischen Analyse auch noch die Reinigungseffizienz auf einzelne Substanzen auswerten.

Bei der Wiederverwertung von Post-Consumer Kunststoffabfällen ist es zweckmäßig, zwischen dem Aufschmelzextruder und dem Entgasungsextruder einen Schmelzefilter zum Entfernen von Fremdkörpern aus der Kunststoffschmelze anzuordnen.

Wenn die Granuliervorrichtung zur Granulierung der Kunststoffschmelze eine Nass-Granuliervorrichtung ist, ist es zweckmäßig, nach der Granuliervorrichtung eine Trocknungseinrichtung zur Trocknung des Granulats, z.B. eine Trocknungszentrifuge, vorzusehen, damit das Granulat der Geruchsentfernungseinheit in weitgehend trockenem Zustand zugeführt wird.

Bevorzugt ist das mit Ozon anzureichernde Prozessgas Luft, wobei auch, gegebenenfalls gefilterte, Umgebungsluft verwendet werden kann.

Es hat sich gezeigt, dass die Ozonkonzentration im angereicherten Prozessgas zumindest 0,1 ppm betragen und vorzugsweise in einem Bereich zwischen 10 ppm und 100 ppm liegen sollte, um ausreichende Geruchsentfernung zu erzielen, ohne die Umwelt nennenswert zu belasten.

Um beste Geruchsentfernungswirkung zu erzielen, sowie gleichzeitig die Ozonbelastung der Umwelt zu minimieren, ist es erfindungsgemäß bevorzugt, eine Einstellvorrichtung zur Einstellung der Ozonmenge, die der Geruchsentfernungseinheit zugeführt wird, in Abhängigkeit von der Ozonkonzentration des Abgasstroms aus der Geruchsentfernungseinheit, vorzusehen. Für die Geruchsentfernungswirkung ist die absolute Ozonmenge entscheidend, also wie viele Moleküle Ozon insgesamt auf das Granulat, und folglich die vorhandenen Geruchsstoffmoleküle, treffen (g Ozon/kg Kunststoff).

Die zugeführte Ozonmenge kann durch die Einstellvorrichtung auf unterschiedliche Weise beeinflusst werden:
- Entweder, indem die Ozonkonzentration des zugeführten Prozessgases verändert wird; oder
- indem die Behandlungszeit (Prozessdauer) verkürzt/gestoppt wird; oder
- indem die Strömungsgeschwindigkeit des Prozessgases manipuliert wird.

Um diese Einstellung möglichst exakt, schnell und automatisiert durchzuführen, sieht die Erfindung ergänzend eine Inlinemesseinrichtung zur Messung der Ozonkonzentration des Abgasstroms aus der Geruchsentfernungseinheit vor, wobei die Inline-Messeinrichtung die Einstellvorrichtung zur Einstellung der der Geruchsentfernungseinheit zugeführten Ozonmenge in Abhängigkeit von der gemessenen Ozonkonzentration steuert. Dadurch wird einerseits ausreichend Ozon produziert bzw. dem Prozessgasstrom zugeführt, um geruchsbildende Stoffe durch Oxidation zu entfernen, gleichzeitig wird durch die Inlinemessung und Regelung die emittierte Menge an Ozon gering gehalten. Die benötigte Ozonkonzentration des zugeführten Prozessgases ist abhängig von der Kontaminationsintensität des Granulats bzw. dessen Geruchsintensität. Ist das Granulat stärker kontaminiert, wird mehr Ozon bei der Behandlung (Reaktion mit den Geruchsstoffen) verbraucht. Am Abgasstrom wird die verbliebene Ozonkonzentration gemessen. Wird ein bestimmter Schwellenwert der Ozonkonzentration im Abgasstrom überschritten, so wird die dem Prozessgas oder als Prozessgas zugeführte Ozonmenge gedrosselt, bis der Schwellenwert der Ozonkonzentration in dem Abgasstrom wieder unterschritten wird.

Mit dieser Regelung können zwei Aufgaben gelöst werden:
a.) Zum einen soll kein überschüssiges Ozon produziert und in Folge in die Umwelt freigesetzt werden.
b.) Zum anderen soll festgestellt werden, wann der Geruchsentfernungsprozess abgeschlossen ist, also die Geruchsstoffe mit dem Ozon abreagiert sind.

Grundsätzlich kann der ablaufende chemische Prozess wie folgt dargestellt werden:

O₃ (Ozoninput) + X → O-X + O₂

wobei:

| | | |
|---|---|---|
| X | ... | Geruchsstoff |
| O-X | ... | oxidierter Geruchsstoff |

Wenn noch genügend Geruchsstoffe als Reaktionspartner für das Ozon vorhanden sind, kann das Ozon vollständig abreagieren und dabei verbraucht werden. Im Abgasstrom finden sich dann die oxidierten Geruchsstoffe und Sauerstoff, woraus man erkennt, dass der Geruchsentfernungsprozess noch nicht abgeschlossen ist.

Wird jedoch mehr Ozon mit dem Prozessgas in die Geruchsentfernungseinheit eingespeist als mit den Geruchsstoffen, mit denen das Granulat in der Geruchsentfernungseinheit beladen ist, reagieren kann, verlässt das Ozon über den Abgasstrom die Geruchsentfernungseinheit und ist dort messbar.

Das lässt sich vereinfacht folgendermaßen darstellen:
n O₃ (Ozoninput) + X → O-X + O₂ + (n-1) O₃ (mittels eines Sensors messbares Ozon im Abgasstrom)
wobei:

| | | |
|---|---|---|
| n ≥ 2 | | |
| X | ... | Geruchsstoff |
| O-X | ... | oxidierter Geruchsstoff |

Dieser Fall tritt ein, wenn zu viel Ozon in die Geruchsentfernungseinheit eingespeist wird. Es sind die nachfolgenden Lösungen für dieses Problems vorgesehen:
Es wird zu Beginn des Prozesses Prozessgas mit niedriger Ozonkonzentration eingespeist. Wird im Abgasstrom kein Ozon gemessen, wird schrittweise die Ozonkonzentration des eingespeisten Prozessgases erhöht, bis im Abgasstrom eine bestimmte Ozonkonzentration (z.B. ein bestimmter Anteil der eingespeisten Menge) vorhanden ist. Dann wird die Ozonkonzentration des zugeführten Prozessgases konstant gehalten, bis der Geruchsentfernungsprozess abgeschlossen ist, also die Geruchsstoffe mit dem Ozon abreagiert sind.

Alternativ oder zusätzlich kann man die Strömungsgeschwindigkeit des Prozessgasstroms verändern, um die Ozonmenge einzustellen. Dazu startet man mit einer bestimmten Flussrate des Prozessgasstroms und misst die Ozonkonzentration im Abgasstrom. Ist dort zu viel Ozon vorhanden, wird Schritt für Schritt die Flussrate reduziert, bis kein Ozon mehr im Abgasstrom messbar ist bzw. ein bestimmter Schwellenwert im Abgasstrom unterschritten wird. Dann wird die Flussrate konstant gehalten, bis der Geruchsentfernungsprozess abgeschlossen ist.

Während des Geruchsentfernungsprozesses läuft die Reaktion im Idealfall so ab, dass das Ozon vollständig verbraucht wird, d.h.:

O₃ (Ozoninput) + X → O-X + O₂

wobei:

| | | |
|---|---|---|
| X | ... | Geruchsstoff |
| O-X | ... | oxidierter Geruchsstoff |

Mit Fortdauer des Geruchsentfernungsprozesses stehen immer weniger Geruchsstoffmoleküle als Reaktionspartner für das Ozon zur Verfügung und es bleibt immer mehr Ozon übrig. Dann kann im Abgasstrom ein Anstieg der Ozonkonzentration gemessen werden.
n O₃ (Ozoninput) + X → O-X + O₂ + (n-1) O₃ (mittels eines Sensors messbares Ozon im Abgasstrom)
wobei:

| | | |
|---|---|---|
| n ≥ 2 | | |
| X | ... | Geruchsstoff |
| O-X | ... | oxidierter Geruchsstoff |

Dass der Geruchsentfernungsprozess abgeschlossen ist, lässt sich daran erkennen, dass ein bestimmter Anstieg der Ozonkonzentration im Abgasstrom gemessen wird, bzw. ein bestimmter Schwellenwert (z.B. ein bestimmter Anteil der zugeführten Ozonkonzentration) überschritten wird. Sobald dies detektiert wird, wird die Ozonzufuhr unmittelbar gestoppt oder nach dem gleichen Prinzip wie oben beschrieben, schrittweise reduziert, indem entweder die Ozonkonzentration im zugeführten Prozessgas reduziert wird oder die Strömungsgeschwindigkeit schrittweise zurückgefahren wird.

Anzumerken ist, dass die obigen Reaktionsgleichungen nur der Illustration dienen und vereinfacht sind, da sie nur einen Fall zeigen, in welchem der Geruchsstoff nur einmalig oxidiert wird. Tatsächlich werden die meisten Geruchsstoffe durch Ozon mehrfach oxidiert. Anders gesagt, es reagieren zwei oder mehr Ozonmoleküle mit einem Geruchsstoffmolekül. Das Prinzip bleibt aber dasselbe.

Die Inlinemesseinrichtung zur Messung der Ozonkonzentration des Abgasstroms aus der Geruchsentfernungseinheit kann auch zur bloßen Messung der Ozonkonzentration vorgesehen sein, beispielsweise um behördliche Umweltauflagen zu erfüllen oder landesspezifische Grenzwerte einzuhalten.

Um eine Umweltbelastung durch Ozon zu minimieren, ist in einer Ausführungsform der Erfindung optional zumindest eine Einrichtung zum zumindest teilweisen Abbau von im Abgasstrom enthaltenen Ozon vorgesehen, wobei die Einrichtung zum Abbau von Ozon bevorzugt zur thermischen oder katalytischen Behandlung des Abgasstroms oder zur Bestrahlung des Abgasstroms mit elektromagnetischen Wellen ausgebildet ist. Die elektromagnetischen Wellen sind vorzugsweise UV-Licht mit einer Wellenlänge von zumindest 254 nm.

Diese Einrichtung zum zumindest teilweisen Abbau von im Abgasstrom enthaltenen Ozon kann optional auf Basis der im Abgasstrom gemessenen Ozonkonzentration gesteuert ein- und ausgeschaltet oder geregelt werden.

Die Erfindung wird nachfolgend anhand beispielhafter Ausführungsformen unter Bezugnahme auf die Zeichnungen näher erläutert.
Fig. 1 zeigt schematisch eine erfindungsgemäße Vorrichtung zur Wiederaufbereitung von Flakes aus zerkleinerten und gewaschenen Post-Consumer Kunststoffabfällen.
Fig. 2 zeigt eine mögliche Ausführungsform eines Details der Vorrichtung von Fig. 1.

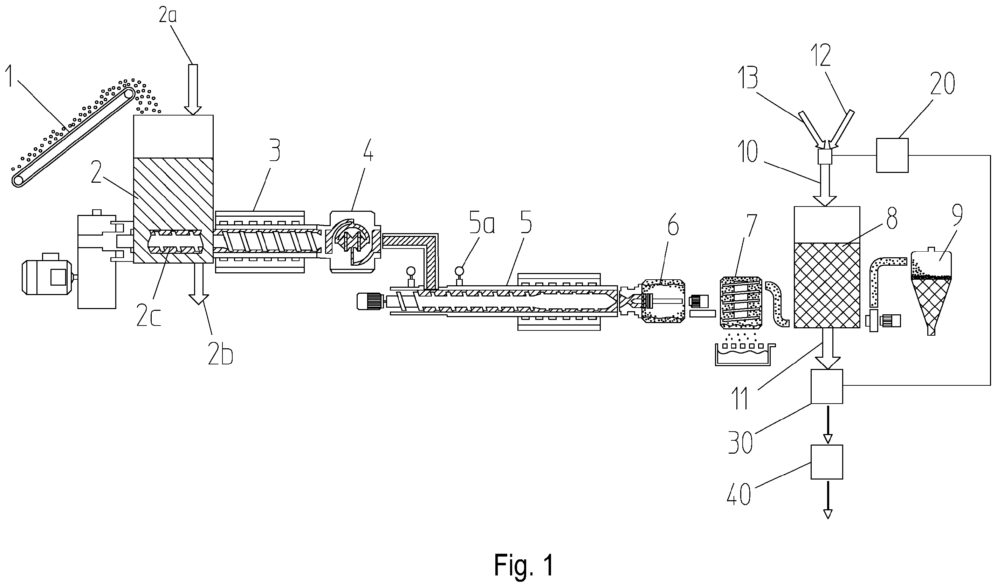

Die in Fig. 1 schematisch dargestellte Vorrichtung zur Wiederaufbereitung von Flakes aus zerkleinerten und gewaschenen Post-Consumer Kunststoffabfällen weist eine Vorbehandlungseinheit 2 zur Trocknung und Homogenisierung der Flakes aus zerkleinerten und gewaschenen Post-Consumer Kunststoffabfällen auf, wobei die Beschickung der Vorbehandlungseinheit 2 mit Flakes durch eine Beschickungseinheit 1 in Form einer Förderschnecke erfolgt. Die Vorbehandlungseinheit 2 weist einen Gaszulauf 2a, der bevorzugt als Heißgaszulauf ausgestaltet ist, und einen Gasablauf 2b auf. Gas, bevorzugt Heißluft, wird durch den Gaszulauf 2a hindurch in die Vorbehandlungseinheit 2 zugeführt, in der sich die Flakes befinden. Mit dem zugeführten Gasstrom werden die Flakes getrocknet und homogenisiert. Der mit Feuchtigkeit und eventuell Kontaminationen beladene Gasstrom wird durch den Gasablauf 2b hindurch ausgetragen. Die Austragung der Flakes aus der Vorbehandlungseinheit erfolgt durch eine Förderschnecke 2c in einen Aufschmelzextruder 3, der stromabwärts von der Vorbehandlungseinheit 2 angeordnet ist. Im Aufschmelzextruder 3 werden die getrockneten und homogenisierten Flakes aus Kunststoffabfällen aufgeschmolzen. Stromabwärts vom Aufschmelzextruder 3 ist ein Schmelzefilter 4 zum Entfernen von Fremdkörpern aus der Kunststoffschmelze angeordnet.

Stromabwärts vom Schmelzefilter 4 befindet sich ein Entgasungsextruder 5 mit einem Vakuumanschluss 5a an eine nicht dargestellte Vakuumquelle zum Entgasen der Kunststoffschmelze, gefolgt von einer Granuliervorrichtung 6 zur Granulierung der Kunststoffschmelze. Das in der Granuliervorrichtung 6 hergestellte Granulat wird in einer Trocknungszentrifuge 7 getrocknet und anschließend einer Geruchsentfernungseinheit 8 zugeführt, wo das Granulat einer Geruchsentfernung unterzogen wird. Nach seiner Geruchsentfernungsbehandlung wird das Granulat aus der Geruchsentfernungseinheit 8 ausgetragen und kann dann entweder direkt verarbeitet oder einem Vorratssilo 9 zugeführt werden.

Die Geruchsentfernungseinheit 8 weist einen Prozessgaszulauf 10 und einen Gasablauf 11 zum Austragen eines Abgasstroms auf, wobei der Prozessgaszulauf 10 der Geruchsentfernungseinheit 8 mit einer Ozonquelle 12, z.B. in Form eines Behälters, der mit einem mit Ozon angereicherten Gas gefüllt ist, z.B. mit Ozon angereicherter Druckluft, oder einer Ozonerzeugungsvorrichtung 13 verbunden ist, wodurch der Geruchsentfernungseinheit 8 Ozon oder mit Ozon angereichertes Prozessgas zuführbar ist.

Eine Einstellvorrichtung 20 dient zur Einstellung der Ozonmenge, die der Geruchsentfernungseinheit 8 zugeführt wird, in Abhängigkeit von der Ozonkonzentration des Abgasstroms aus der Geruchsentfernungseinheit 8. Die Einstellung der zuzuführenden Ozonmenge erfolgt beispielsweise durch ein von der Einstellvorrichtung 20 betätigtes Regelventil im Prozessgaszulauf 10. Die Einstellvorrichtung 20 kann als elektronischer Regler mit einem Mikroprozessor, einem Arbeitsspeicher, einem Programmspeicher, in dem ein Regelalgorithmus abgespeichert ist, und Interfaces zur Kommunikation und zur Betätigung von Aktuatoren, wie z.B. dem genannten Regelventil ausgeführt sein. Die Ozonerzeugungsvorrichtung 13 kann so ausgeführt sein, dass sie die Umgebungsluft unter elektrische Spannung setzt, sodass der Sauerstoff in der Umgebungsluft zu Ozon reagiert. Je höher die elektrische Spannung, desto mehr Ozon entsteht. So kann durch Regelung der elektrischen Spannung die Ozonkonzentration des die Ozonerzeugungsvorrichtung 13 verlassenden Gases geregelt werden. Die Regelung der Ozonerzeugungsvorrichtung 13 zur Erzeugung und Variierung der elektrischen Spannung kann durch die Einstellvorrichtung 20 erfolgen.

Alternativ dazu kann die Ozonerzeugungsvorrichtung 13 auf Basis von Bestrahlung von angesaugter Umgebungsluft mit UV-Licht, vorzugsweise mit einer Wellenlänge kleiner als 240 nm erfolgen. Bei einer solchen Ozonerzeugungsvorrichtung 13 wird die Ozonkonzentration des die Ozonerzeugungsvorrichtung 13 verlassenden Gases geregelt, indem die Wellenlänge des UV-Lichtes verändert wird. Auch diese Regelung kann mit der Einstellvorrichtung 20 erfolgen. Da UV-Licht kürzerer Wellenlänge energiereicher ist, wird damit mehr Ozon erzeugt als bei Bestrahlung mit UV-Licht längerer Wellenlänge.

Bevorzugt wird die Einstellvorrichtung 20 durch eine Inlinemesseinrichtung 30 zur Messung der Ozonkonzentration des Abgasstroms aus der Geruchsentfernungseinheit 8 gesteuert, wobei die Steuerung der Einstellvorrichtung 20 zur Einstellung der der Geruchsentfernungseinheit 8 zugeführten Ozonmenge in Abhängigkeit von der gemessenen Ozonkonzentration erfolgt. Mit einer solchen Konfiguration sind die oben beschriebenen Prozesse zur Geruchsentfernung bei gleichzeitig geringer Ozonbelastung der Umgebung durchführbar. Die Inlinemesseinrichtung 30 kann direkt im oder am Gasablauf 11 angeordnet sein, oder alternativ dazu vom Gasablauf 11 entfernt angeordnet sein, aber über einen Ozonsensor verfügen, der in den Gasablauf 11 eingebaut ist.

Am Gasablauf der Geruchsentfernungseinheit 8 ist optional eine Einrichtung 40 zum zumindest teilweisen Abbau von im Abgasstrom enthaltenen Ozon vorgesehen, wobei die Einrichtung 40 zum Abbau von Ozon bevorzugt zur thermischen oder katalytischen Behandlung des Abgasstroms oder zur Bestrahlung des Abgasstroms mit elektromagnetischen Wellen ausgebildet ist. Wenn der Abbau von Ozon durch Bestrahlung des abgezogenen Prozessgases mit elektromagnetischen Wellen erfolgt, so wird vorzugsweise UV-Licht mit einer Wellenlänge von zumindest 254 nm verwendet.

Aufgrund seiner einfachen Handhabbarkeit ist das mit Ozon anzureichernde Prozessgas bevorzugt Luft. Die Ozonkonzentration im angereicherten Prozessgas sollte zumindest 0,1 ppm betragen und vorzugsweise in einem Bereich zwischen 10 ppm und 100 ppm liegen.

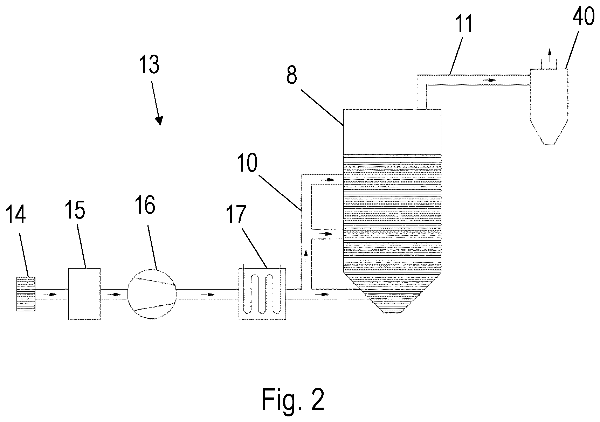

In Fig. 2 ist schematisch eine erfindungsgemäße Ausführungsform der Ozongaserzeugungsvorrichtung 13 dargestellt, die aus der Umgebungsluft Ozon gewinnt. Diese Ozongaserzeugungsvorrichtung 13 umfasst einen Luftfilter 14, der mit der Umgebungsluft in Verbindung steht und Staub, etc. aus der Umgebungsluft ausfiltert. Die solcherart gereinigte Umgebungsluft wird einem Ozongenerator 15 zugeführt, der nach dem Fachmann bekannten Prinzipien einen Teil der Umgebungsluft in Ozon umwandelt und dadurch mit Ozon angereicherte Luft als Prozessgas erzeugt. Der Ausgang des Ozongenerators 15 ist mit dem Eingang, d.h. der Ansaugseite, eines Gebläses 16 verbunden. Das Gebläse 16 bewirkt einerseits die Ansaugung von Luft durch den Luftfilter 14 und den Ozongenerator 15 hindurch, und bläst andererseits das Prozessgas durch eine Heizung 17 in den Prozessgaszulauf 10 und durch den Prozessgaszulauf 10 hindurch in die Geruchsentfernungseinheit 8. Das Prozessgas, dessen Ozon-Anteil durch die Geruchsentfernung verbraucht worden ist, verlässt als Abgasstrom die Geruchsentfernungseinheit 8 durch den Gasablauf 11 und gelangt in die Einrichtung 40 zum Abbau von Ozon, wo überschüssiges Ozon reduziert wird. Angemerkt wird, dass der Begriff "Ozongaserzeugungsvorrichtung" nicht dahingehend zu verstehen ist, dass diese Vorrichtung reines Ozon erzeugt. Vielmehr ist mit dem Begriff "Ozongaserzeugungsvorrichtung" gemeint, dass die Vorrichtung mit Ozon angereichertes Gas, insbesondere mit Ozon angereicherte Luft erzeugt.

Im Rahmen einer Versuchsreihe wurde der Prozessgasstrom zur Geruchsentfernungseinheit 8 mit 35 ppm Ozon angereichert und in der Geruchsentfernungseinheit 8 wurden Granulate aus Post-Consumer Abfällen unterschiedlicher Kunststoffart jeweils 2 - 2,7 h lang mit dem mit Ozon angereicherten Prozessgas durchströmt. Bei einer Ozonkonzentration von 35 ppm und einer Flussrate von 1600 m³ Prozessgas/h betrug die geförderte Ozonmenge 120g/h. Umgerechnet wurden somit 0,24g Ozon/kg Kunststoff zugeführt.

Im Anschluss daran wurden in einer Blindstudie jeweils zwei Proben gleichen Kunststoffmaterials, wovon eine Probe auf herkömmliche Weise 2 - 2,7h mit Heißluft und die andere Probe gleich lang und bei gleicher Temperatur mit 35 ppm Ozon enthaltender Heißluft als Prozessgas durchströmt wurde, einem Gremium von ausgebildeten Geruchstestern zur Bewertung zur Verfügung gestellt. Dabei wurden die mit Ozon behandelten Granulate aus HDPE, PP und LDPE bezüglich ihrer Geruchsintensität und Hedonik von den Testern signifikant besser bewertet als das ausschließlich mit Heißluft behandelte Vergleichsmaterial.

## Patentansprüche

1. Vorrichtung zur Wiederaufbereitung von Flakes aus zerkleinerten und gewaschenen Post-Consumer Kunststoffabfällen, umfassend:
eine Vorbehandlungseinheit (2) zur Trocknung und Homogenisierung der Flakes aus zerkleinerten und gewaschenen Post-Consumer Kunststoffabfällen,
einen stromabwärts von der Vorbehandlungseinheit (2) angeordneten Aufschmelzextruder (3) zum Aufschmelzen der in der Vorbehandlungseinheit (2) getrockneten und homogenisierten Kunststoffabfälle,
einen stromabwärts vom Aufschmelzextruder (3) angeordneten Entgasungsextruder (5) mit einem Anschluss (5a) an eine Vakuumquelle zum Entgasen der Kunststoffschmelze, gegebenenfalls einen zwischen dem Aufschmelzextruder (3) und dem Entgasungsextruder (5) angeordneten Schmelzefilter (4) zum Entfernen von Fremdkörpern aus der Kunststoffschmelze,
eine stromabwärts vom Entgasungsextruder (5) angeordnete Granuliervorrichtung (6) zur Granulierung der Kunststoffschmelze,
eine stromabwärts von der Granuliervorrichtung (6) angeordnete Geruchsentfernungseinheit (8), um das Granulat einer Geruchsentfernung zu unterziehen,
und gegebenenfalls eine zwischen der Granuliervorrichtung (6) und der Geruchsentfernungseinheit (8) angeordnete Trocknungseinrichtung (7) zur Trocknung des Granulats,
**dadurch gekennzeichnet, dass**
die Geruchsentfernungseinheit (8) einen Prozessgaszulauf (10) und einen Gasablauf (11) zum Austragen eines Abgasstroms aufweist, wobei der Prozessgaszulauf (10) der Geruchsentfernungseinheit (8) mit einer Ozonquelle (12) oder einer
Ozonerzeugungsvorrichtung (13) verbunden ist, wodurch der Geruchsentfernungseinheit (8) mit Ozon angereichertes Prozessgas zuführbar ist.

2. Vorrichtung nach Anspruch 1, **gekennzeichnet durch** eine Inlinemesseinrichtung (30) zur Messung der Ozonkonzentration des Abgasstroms aus der Geruchsentfernungseinheit (8).

3. Vorrichtung nach Anspruch 1 oder 2, **gekennzeichnet durch** eine Einstellvorrichtung (20) zur Einstellung der Ozonmenge, die der Geruchsentfernungseinheit (8) zugeführt wird, in Abhängigkeit von der Ozonkonzentration des Abgasstroms aus der Geruchsentfernungseinheit.

4. Vorrichtung nach einem der vorhergehenden Ansprüche, **dadurch gekennzeichnet, dass** die Vorrichtung zumindest eine Einrichtung (40) zum zumindest teilweisen Abbau von im Abgasstrom enthaltenen Ozon aufweist, wobei die Einrichtung (40) zum Abbau von Ozon bevorzugt zur thermischen oder katalytischen Behandlung des Abgasstroms oder zur Bestrahlung des Abgasstroms mit elektromagnetischen Wellen ausgebildet ist.

5. Vorrichtung nach einem der vorhergehenden Ansprüche, **dadurch gekennzeichnet, dass** das mit Ozon anzureichernde Prozessgas Luft ist.

6. Vorrichtung nach einem der vorhergehenden Ansprüche, **dadurch gekennzeichnet, dass** die Ozonkonzentration im angereicherten Prozessgas zumindest 0,1 ppm beträgt und vorzugsweise in einem Bereich zwischen 10 ppm und 100 ppm liegt.

7. Verfahren zur Wiederaufbereitung von Flakes aus zerkleinerten und gewaschenen Post-Consumer Kunststoffabfällen, umfassend:
das Trocknen und Homogenisieren der Flakes aus zerkleinerten und gewaschenen Post-Consumer Kunststoffabfälle in einem Gasstrom, insbesondere einem Heißgasstrom,
das Aufschmelzen der getrockneten und homogenisierten Kunststoffabfälle zu einer Kunststoffschmelze unter Verwendung eines Aufschmelzextruders,
optional das Ausfiltern von Fremdkörpern aus der Kunststoffschmelze,
das Entgasen der Kunststoffschmelze unter Verwendung eines Entgasungsextruders,
das Granulieren der Kunststoffschmelze,
optional das Trocknen des Granulats,
das Entfernen von Gerüchen aus dem Granulat durch einen Prozessgasstrom,
**gekennzeichnet durch**
das Anreichern des zum Entfernen von Gerüchen aus dem Granulat verwendeten Prozessgasstroms mit Ozon oder das Bereitstellen des Prozessgasstroms als ein mit Ozon angereichertes Gas.

8. Verfahren nach Anspruch 7, **dadurch gekennzeichnet, dass** die Ozonmenge, die dem Granulat beim Entfernen der Gerüche aus dem Granulat zugeführt wird, in Abhängigkeit von der Ozonkonzentration des Prozessgases beim Abziehen nach der Geruchsentfernungsbehandlung des Granulats eingestellt wird.

9. Verfahren nach Anspruch 8, **dadurch gekennzeichnet, dass** die Ozonkonzentration des Prozessgases beim Abziehen nach der Geruchsentfernungsbehandlung des Granulats durch Inlinemessungen erfasst wird und die Einstellung der dem Granulat beim Entfernen der Gerüche aus dem Granulat zugeführten Ozonmenge in Abhängigkeit von der gemessenen Ozonkonzentration erfolgt.

10. Verfahren nach einem der Ansprüche 7 bis 9, **dadurch gekennzeichnet, dass** das im abgezogenen Prozessgas enthaltene Ozon zumindest teilweise abgebaut wird, wobei der Abbau von Ozon bevorzugt durch thermische oder katalytische Behandlung des abgezogenen Prozessgases erfolgt.

11. Verfahren nach einem der Ansprüche 7 bis 9, **dadurch gekennzeichnet, dass** das im abgezogenen Prozessgas enthaltene Ozon zumindest teilweise abgebaut wird, wobei der Abbau von Ozon durch Bestrahlung des abgezogenen Prozessgases mit elektromagnetischen Wellen erfolgt, wobei die elektromagnetischen Wellen vorzugsweise UV-Licht mit einer Wellenlänge von zumindest 254 nm sind.

12. Verfahren nach einem der Ansprüche 7 bis 11, **dadurch gekennzeichnet, dass** das mit Ozon anzureichernde Prozessgas Luft ist.

13. Verfahren nach einem der Ansprüche 7 bis 12, **dadurch gekennzeichnet, dass** die Ozonkonzentration im angereicherten Prozessgas zumindest 0,1 ppm beträgt und vorzugsweise in einem Bereich zwischen 10 ppm und 100 ppm liegt.

## Claims

1. A device for recycling flakes from shredded and washed post-consumer plastic waste, comprising:
a pre-treatment unit (2) for drying and homogenising the flakes from shredded and washed post-consumer plastic waste,
a melting extruder (3) arranged downstream of the pre-treatment unit (2) for melting the plastic waste dried and homogenized in the pre-treatment unit (2),
a degassing extruder (5) arranged downstream of the melting extruder (3) and having a connection (5a) to a vacuum source for degassing the plastic melt,
optionally a melt filter (4) arranged between the melting extruder (3) and the degassing extruder (5) for removing foreign matter from the plastic melt,
a granulating device (6) arranged downstream of the degassing extruder (5) for granulating the plastic melt,
an odour removal unit (8) arranged downstream of the granulating device (6) for subjecting the granules to odour removal,
and optionally a drying device (7) arranged between the granulating device (6) and the odour removal unit (8) for drying the granules,
**characterised in that**
the odour removal unit (8) has a process gas feed (10) and a gas discharge (11) for discharging an exhaust gas stream, wherein the process gas feed (10) of the odour removal unit (8) is connected to an ozone source (12) or an ozone generating device (13), whereby the odour removal unit (8) may be supplied with process gas enriched with ozone.

2. A device according to claim 1, **characterised by** an in-line measuring device (30) for measuring the ozone concentration of the exhaust gas stream from the odour removal unit (8).

3. A device according to claim 1 or 2, **characterised by** an adjustment device (20) for adjusting the amount of ozone, which is supplied to the odour removal unit (8), in dependency on the ozone concentration of the exhaust gas stream from the odour removal unit.

4. A device according to any of the preceding claims, **characterised in that** the device has at least one apparatus (40) for the at least partial depletion of ozone contained in the exhaust gas stream, wherein the apparatus (40) is configured to deplete ozone preferably for the thermal or catalytic treatment of the exhaust gas stream or for irradiating the exhaust gas stream using electromagnetic waves.

5. A device according to any of the preceding claims, **characterised in that** the process gas to be enriched with ozone is air.

6. A device according to any of the preceding claims, **characterised in that** the ozone concentration in the enriched process gas is at least 0.1 ppm and is preferably in a range between 10 ppm and 100 ppm.

7. A method for recycling flakes from shredded and washed post-consumer plastic waste, comprising:
drying and homogenising the flakes from shredded and washed post-consumer plastic waste in a gas stream, in particular a hot gas stream,
melting the dried and homogenized plastic waste into a plastic melt using a melting extruder, optionally filtering out foreign matter from the plastic melt,
degassing the plastic melt using a degassing extruder,
granulating the plastic melt,
optionally drying the granules,
removing odour from the granules by way of a process gas stream,
**characterised by**
enriching the process gas stream used for removing odour from the granules with ozone or providing the process gas stream as a gas enriched with ozone.

8. A method according to claim 7, **characterised in that** the amount of ozone, which is supplied to the granules when removing the odour from the granules, is adjusted in dependency on the ozone concentration of the process gas upon removal following the odour removal treatment of the granules.

9. A method according to claim 8, **characterised in that** the ozone concentration of the process gas upon removal following the odour removal treatment of the granules is detected by way of in-line measurements and that the adjustment of the amount of ozone supplied to the granules when removing the odour from the granules is carried out in dependency on the ozone concentration measured.

10. A method according to any of the claims 7 to 9, **characterised in that** the ozone contained in the removed process gas is depleted at least in part, wherein the depletion of ozone is preferably done by way of thermal or catalytic treatment of the process gas removed.

11. A method according to any of the claims 7 to 9, **characterised in that** the ozone contained in the removed process gas is depleted at least in part, wherein the depletion of ozone is done by way of irradiation of the removed process gas using electromagnetic waves, wherein the electromagnetic waves are preferably UV light having a wavelength of at least 254 nm.

12. A method according to any of the claims 7 to 11, **characterised in that** the process gas to be enriched with ozone is air.

13. A method according to any of the claims 7 to 12, **characterised in that** the ozone concentration in the enriched process gas is at least 0.1 ppm and lies preferably in a range between 10 ppm and 100 ppm.

## Revendications

1. Dispositif de retraitement de paillettes issues de déchets plastiques post-consommation broyés et lavés, comprenant:
une unité de prétraitement (2) pour le séchage et l'homogénéisation des paillettes issues de déchets plastiques post-consommation broyés et lavés,
une extrudeuse à fusion (3) disposée en aval de l'unité de prétraitement (2) pour faire fondre les déchets plastiques séchés et homogénéisés dans l'unité de prétraitement (2),
une extrudeuse de dégazage (5) disposée en aval de l'extrudeuse à fusion (3) et comprenant un raccordement (5a) à une source de vide pour dégazer la matière plastique fondue,
éventuellement un filtre de matière fondue (4) disposé entre l'extrudeuse à fusion (3) et l'extrudeuse de dégazage (5) pour éliminer les particules étrangères de la matière plastique fondue,
un dispositif de granulation (6) disposé en aval de l'extrudeuse de dégazage (5) pour granuler la matière plastique fondue,
une unité d'élimination des odeurs (8) disposée en aval du dispositif de granulation (6) pour soumettre les granulés à un processus d'élimination des odeurs, et
éventuellement un dispositif de séchage (7) disposé entre le dispositif de granulation (6) et l'unité d'élimination des odeurs (8) pour le séchage des granulés,
**caractérisé en ce que** l'unité d'élimination des odeurs (8) présente une entrée de gaz de procédé (10) et une sortie de gaz (11) pour l'évacuation d'un flux de gaz d'échappement, l'entrée de gaz de procédé (10) de l'unité d'élimination des odeurs (8) étant reliée à une source d'ozone (12) ou à un dispositif de génération d'ozone (13), permettant ainsi d'alimenter un gaz de procédé enrichi en ozone dans l'unité d'élimination des odeurs (8).

2. Dispositif selon la revendication 1, **caractérisé par** un dispositif de mesure en ligne (30) permettant de mesurer la concentration d'ozone du flux de gaz d'échappement provenant de l'unité d'élimination des odeurs (8).

3. Dispositif selon la revendication 1 ou 2, **caractérisé par** un dispositif de réglage (20) permettant d'ajuster la quantité d'ozone ajoutée à l'unité d'élimination des odeurs (8) en fonction de la concentration d'ozone dans le flux de gaz d'échappement provenant de l'unité d'élimination des odeurs.

4. Dispositif selon l'une quelconque des revendications précédentes, **caractérisé en ce que** le dispositif comprend au moins un dispositif (40) permettant de dégrader au moins partiellement l'ozone contenu dans le flux de gaz d'échappement, le dispositif (40) de dégradation de l'ozone étant de préférence conçu pour un traitement thermique ou catalytique du flux de gaz d'échappement, ou pour l'irradiation du flux de gaz d'échappement avec des ondes électromagnétiques.

5. Dispositif selon l'une quelconque des revendications précédentes, **caractérisé en ce que** le gaz de procédé à enrichir en ozone est de l'air.

6. Dispositif selon l'une quelconque des revendications précédentes, **caractérisé en ce que** la concentration d'ozone dans le gaz de procédé enrichi est d'au moins 0,1 ppm et, de preference, est comprise entre 10 ppm et 100 ppm.

7. Procédé de retraitement de paillettes issues de déchets plastiques post-consommation broyés et lavés, comprenant:
sécher et homogénéiser les paillettes issues de déchets plastiques post-consommation broyés et lavés dans un flux de gaz, notamment un flux de gaz chaud,
faire fondre les déchets plastiques séchés et homogénéisés en une matière plastique fondue en utilisant une extrudeuse à fusion,
éventuellement filtrer des particules étrangères de la matière plastique fondue,
dégazer la matière plastique fondue en utilisant une extrudeuse de dégazage,
granuler la matière plastique fondue,
éventuellement sécher les granulés,
éliminer des odeurs des granulés par l'intermédiaire d'un flux de gaz de procédé,
**caractérisé par** l'enrichissement en ozone du flux de gaz de procédé utilisé pour l'élimination des odeurs des granulés, ou par la fourniture du flux de gaz de procédé sous forme de gaz enrichi en ozone.

8. Procédé selon la revendication 7, **caractérisé en ce que** la quantité d'ozone ajoutée aux granulés lors de l'élimination des odeurs des granulés est ajustée en fonction de la concentration d'ozone du gaz de procédé lors du soutirage après le traitement pour l'élimination des odeurs des granulés.

9. Procédé selon la revendication 8, **caractérisé en ce que** la concentration d'ozone du gaz de procédé est détectée par mesure en ligne lors du soutirage après le traitement pour l'élimination des odeurs des granulés, et le réglage de la quantité d'ozone ajoutée aux granulés lors de l'élimination des odeurs des granulés est réalisé en fonction de la concentration d'ozone mesurée.

10. Procédé selon l'une quelconque des revendications 7 à 9, **caractérisé en ce que** l'ozone contenu dans le gaz de procédé soutiré est au moins partiellement dégradé, la dégradation de l'ozone étant de préférence réalisée par traitement thermique ou catalytique du gaz de procédé soutiré.

11. Procédé selon l'une quelconque des revendications 7 à 9, **caractérisé en ce que** l'ozone contenu dans le gaz de procédé soutiré est au moins partiellement dégradé, la dégradation de l'ozone étant réalisée par irradiation du gaz de procédé soutiré avec des ondes électromagnétiques, les ondes électromagnétiques de préférence étant des rayons UV d'une longueur d'onde d'au moins 254 nm.

12. Procédé selon l'une quelconque des revendications 7 à 11, **caractérisé en ce que** le gaz de procédé à enrichir en ozone est de l'air.

13. Procédé selon l'une quelconque des revendications 7 à 12, **caractérisé en ce que** la concentration d'ozone dans le gaz de procédé enrichi est d'au moins 0,1 ppm et, de preference, est comprise entre 10 ppm et 100 ppm.
